# EUROPEAN PATENT APPLICATION

(11) **EP 1 839 648 A2**
(43) Date of publication of application: **03.10.2007**
(21) Application number: 07001365.1
(22) Date of filing: 12.11.1999
(51) Int. Cl.: A61K 9/127, A61K 9/12, A61K 47/24, A61K 47/28, A61P 11/06, A61P 31/08, A61P 9/00, A61P 9/12, A61P 19/08, A61P 19/10, A61P 29/00, A61P 31/10, A61P 1/08, A61P 3/10

(54) **An inhalation system**

(30) Priority: 12.11.1998 US 108126 P; 12.11.1998 US 108067 P
(62) Divisional of application: 99958945.0
(71) Applicant: Transave, Inc., New Jersey 08550 (US)
(72) Inventor: Pilkiewicz, Frank G, Princeton Junction, NJ 08500 (US)
(74) Representative: Mallalieu, Catherine Louise

(57) **Abstract**

An inhalation system of a composition having lipids and a bioactive agent and an inhalation device.

## Description

The present invention relates to a system for administering pharmaceuticals by inhalation. More particularly the present invention relates to the use of lipids as part of the system.

The pulmonary system is subject to many disorders. There is a continuing need to treat lung diseases such as pre-cancerous or cancerous conditions, damage caused by tobacco smoke and other environmental insults, inflammations and infections.

The lungs can also be a portal to the body by means of uptake by cells of the lung such as aveolar macrophages or through the lymphatic system. Administration of drugs through the lung portal for systematic treatment can avoid hepatic first pass inactivation and allow for lower doses with fewer side effects.

Lung disease is the number three killer in the United States, responsible for one in seven deaths. Significant lung diseases includes such illnesses as: asthma, lung cancer, chronic obstructive pulmonary disease (COPD, which includes emphysema and chronic bronchitis), influenza, pneumonia, tuberculosis, respiratory distress syndrome (RDS), cystic fibrosis, sudden infant death syndrome (SIDS), respiratory synctial virus (RSV), AIDS related lung diseases and sarcoidosis. Every year approximately 335,000 Americans die of lung disease. Lung disease not only causes death, but can result in chronic conditions such as asthma, emphysema and chronic bronchitis.

In the current treatment of lung disease both injectable and pulmonary (via inhalation) administered drugs are employed. In comparison to injection, the administration of a drug by inhalation to treat lung disease is attractive. Inhalation is a more localized administration of the lung disease therapeutic and, therefore, can be more effective. Inhalation can be easier to use. In certain instances the therapeutic can be self-administered leading to better patient compliance and reduced cost. Although inhalation of therapeutics appear to be an attractive alternative to injection for treating lung disease, inhalation administration still has several significant disadvantages: (1) due to the immunology of the lung, drugs that are administered by inhalation quickly clear the lung and, therefore, yield short term therapeutic effects. This rapid clearance can result in the drug having to be administered more frequently and, therefore, adversely affecting patient compliance and increasing the risk of side effects; (2) targeted delivery of the drug by inhalation to the site of disease is not possible, and the therapeutic is treated like a foreign particle that is quickly cleared from the lung, and eventually it ends up in the reticuloendethial system; (3) inhalation formulations are susceptible to both chemical and enzymatic in-vivo degradation. This degradation is particularly detrimental to peptide and protein formulations; and (4) due to aggregation and lack of stability, formulations of high molecular weight compounds like peptides and proteins are not effectively administered as aerosols, nebulized sprays or as dry powder formulations.

The present invention can overcome these disadvantages in lung disease inhalation therapy, and more importantly it offers new advantages to inhalation that can enhance the therapeutic index of a currently used inhalation or injectable lung disease drug. The invention can be used for the successful entrapment and delivery of both low and high molecular weight compounds. The present invention provides for lipid-containing bioactive agents which can be administered by inhalation as part of a delivery system.

### Summary of the Invention

A system for administering a bioactive compound by inhalation having:
(a) a composition comprising the biologically-active compound and a lipid mixture; and
(b) an inhalation delivery device;
   wherein the lipid mixture comprises:
   (1) a first mixture comprising phosphatidylcholine, negatively-charged lipid, and sterol in the molar ratio of 1-20:1-10:0.5-5;
   (2) a second mixture comprising phosphatidylcholine, negatively-charged lipid and albumin in the molar ratio of 1-20:1-10:0.1-10;
   (3) a third mixture comprising phosphatidylcholine, positively-charged lipid and phosphatidylethanolamine in the molar ratio of 1-20:0.5-20:1-10;
   (4) a fourth lipid mixture having phosphatidylcholines: negatively-charged lipids: phosphatidylethanolamines in the molar ratio of 1-20:1-10:1-10;
   (5) a fifth lipid mixture having phosphatidylcholines: positively-charged lipids: albumin compounds in the molar ratio of 1-20:0.5-20:0.1-10; or
   (6) a sixth lipid mixture having phosphatidylcholines: positively-charged lipids: sterol compounds in the molar ratio of 1-20:0.5-20:0.5-5.
   wherein the bioactive agent lipid mixture weight ratio is from 10:1 to 1:500.

The present invention also includes the system wherein the lipid mixture includes:
(i) phosphatidylcholines: negatively-charged lipids: sterol compounds: phosphatidylethanolamines of the molar ratios 1-20:1-10:0.5-5:1-10;
(ii) phosphatidylcholines: negatively-charged lipids: sterol compounds: albumin compounds in the molar ratio of 1-20:1-10:0.5-5:0.1-10;
(iii) phosphatidylcholines: negatively-charged lipids: sterol compounds: phosphatidylethanolamines: albumin compounds in the molar ratio of 1-20:1-10:0.5-5:1-10:0.1-10;
(iv) phosphatidylcholines: negatively-charged lipids: albumin compounds: sterol compounds in the molar ratio of 1-20:1-10:0.1-10:0.5-5;
(v) phosphatidylcholines: positively-charged lipids: phosphatidylethanolamines: sterol compounds in the molar ratio of 1-20:0.5-20:1-10:0.5-5;
(vi) phosphatidylcholines: positively-charged lipids: phosphatidylethanolamines: albumin compounds in the molar ratio of 1-20:0.5-20:1-10:0.1-10;
(vii) phosphatidylcholines: positively-charged lipids: phosphatidylethanolamines: sterol compounds: albumin compounds in the molar ratio of 1-20:0.5-20:1-10:0.5-5:0.1-10; or
(viii) phosphatidylcholines: positively-charged lipids: albumin compounds: sterol compounds in the molar ratio of 1-20:0.5-20:0.1-10:0.5-5.

The present invention includes a method wherein the system is employed for the treatment of a medical condition.

### Detailed Description of the Invention

This invention is an inhalation system for the administration of compositions having lipids and bioactive agents and its use in the treatment of diseases, particularly lung disease. The compositions can include liposomes, lipid complexes, lipid clathrates and proliposomes, i.e., compositions which can form liposomes in vitro or in vivo when contacted with water. Compositions are preferably adopted for use by inhalation, and more preferably for use in an inhalation delivery device for the composition's administration. The inhalation system can be used for the treatment of diseases in both man and animal, particularly lung disease.

The terms "bioactive agent" and "biologically-active" are used interchangeably throughout the specification to describe materials, such as therapeutic, diagnostic and imaging agents, which exhibit biological activity or which can be used for imaging or diagnostic purposes, and which can be administered to living organisms, especially animals such as mammals, particularly humans. Bioactive agents include, but are not limited to: vitamins, hormones, antimetabolites and antimicrobial agents, such as antifungal agents, including polyene antifungals, antibacterial agents, including aminoglycosides, antiviral agents and antiparasitic agents. Bioactive agents also include proteins, peptides, ribo- and deoxyribonucleic acids, nucleotides, nucleosides, oligonucleotides, antihistaminic agents and neuropharmacologic agents, including sedatives. Bioactive agents further include steroidal and nonsteroidal anti-inflammatory agents, diuretic agents, antihypertensive agents, anti-arrhythmic agents, immunogens, immunomodulators, contraceptive agents, antiviral agents, vascular dilating agents, salicylic acid, resorcinol, phenol, retinoic acid, benzodiazepines, antipyretic agents, antispasmodics, anti-pruritic agents, sympathomimetics, decongestants, tranquilizers, anti-spasmodics, anti-emetics, sedatives and hypnotics, steroidal agents, progestational agents, local anesthetics and desensitizing agents, for example antigens and vaccines. Bioactive agents include vitamins, nutrients, such as amino acids, essential fats and minerals, retinoids and anti-neoplastic agents, including the anthracyclines and certain alkylating agents. Bioactive agents also include radiocontrast agents, such as the iodinated radiocontrast agents, for example, iotrolan, NMR contrast agents, radioisotopes, radiolabels and dyes. The above-listed group of bioactive agents, among other agents, including their pharmaceutically acceptable salts, are contemplated for use in the present invention. Determination of compatibilities of the above listed agents with, and the amounts to be utilized in, compositions of the present invention are within the purview of the ordinarily skilled artisan to determine given the teachings of this invention.

The lipids used in the compositions of the present invention can be synthetic, semi-synthetic or naturally-occurring lipids, including phospholipids, tocopherols, sterols, fatty acids, glycoproteins such as albumin, negatively-charged lipids and cationic lipids. In terms of phosholipids, they could include such lipids as egg phosphatidylcholine (EPC), egg phosphatidylglycerol (EPG), egg phosphatidylinositol (EPI), egg phosphatidylserine (EPS), phosphatidylethanolamine (EPE), and phosphatidic acid (EPA); the soya counterparts, soy phosphatidylcholine (SPC); SPG, SPS, SPI, SPE, and SPA; the hydrogenated egg and soya counterparts (e.g., HEPC, HSPC), other phospholipids made up of ester linkages of fatty acids in the 2 and 3 of glycerol positions containing chains of 12 to 26 carbon atoms and different head groups in the 1 position of glycerol that include choline, glycerol, inositol, serine, ethanolamine, as well as the corresponding phosphatidic acids. The chains on these fatty acids can be saturated or unsaturated, and the phospholipid may be made up of fatty acids of different chain lengths and different degrees of unsaturation. In particular, the compositions of the formulations can include DPPC, a major constituent of naturally-occurring lung surfactant. Other examples include dimyristoylphosphatidylcholine (DMPC) and dimyristoylphosphatidylglycerol (DMPG) dipalmitoylphosphatidylcholine (DPPC) and dipalmitoylphosphatidylglycerol (DPPG) distearoylphosphatidylcholine (DSPC) and distearoylphosphatidylglycerol (DSPG), dioleylphosphatidyl-ethanolamine (DOPE) and mixed phospholipids like palmitoylstearoylphosphatidyl-choline (PSPC) and palmitoylstearolphosphatidylglycerol (PSPG), and single acylated phospholipids like mono-oleoyl-phosphatidylethanolamine (MOPE).

The sterols can include, cholesterol, esters of cholesterol including cholesterol hemi-succinate, salts of cholesterol including cholesterol hydrogen sulfate and cholesterol sulfate, ergosterol, esters of ergosterol including ergosterol hemi-succinate, salts of ergosterol including ergosterol hydrogen sulfate and ergosterol sulfate, lanosterol, esters of lanosterol including lanosterol hemi-succinate, salts of lanosterol including lanosterol hydrogen sulfate and lanosterol sulfate. The tocopherols can include tocopherols, esters of tocopherols including tocopherol hemi-succinates, salts of tocopherols including tocopherol hydrogen sulfates and tocopherol sulfates. The term "sterol compound" includes sterols, tocopherols and the like.

The cationic lipids used can include ammonium salts of fatty acids, phospholids and glycerides. The fatty acids include fatty acids of carbon chain lengths of 12 to 26 carbon atoms that are either saturated or unsaturated. Some specific examples include: myristylamine, palmitylamine, laurylamine and stearylamine, dilauroyl ethylphosphocholine (DLEP), dimyristoyl ethylphosphocholine (DMEP), dipalmitoyl ethylphosphocholine (DPEP) and distearoyl ethylphosphocholine (DSEP), N-(2, 3- di-(9-(Z)-octadecenyloxy)-prop-1-yl-N, N, N-trimethylammonium chloride (DOTMA) and 1, 2-bis(oleoyloxy)-3-(trimethylammonio)propane (DOTAP).

The negatively-charged lipids which can be used include phosphatidyl-glycerols (PGs), phosphatidic acids (PAs), phosphatidylinositols (PIs) and the phosphatidyl serines (PSs). Examples include DMPG, DPPG, DSPG, DMPA, DPPA, DSPA, DMPI, DPPI, DSPI, DMPS, DPPS and DSPS.

Phosphatidylcholines, such as DPPC, aid in the uptake by the cells in the lung (e.g., the alveolar macrophages) and helps to sustain release of the bioactive agent in the lung. The negatively charged lipids such as the PGs, PAs, PSs and PIs, in addition to reducing particle aggregation, are believed to play a role in the sustained release characteristics of the inhalation formulation as well as in the transport of the formulation across the lung (transcytosis) for systemic uptake. The sterol compounds are believed to affect the release characteristics of the formulation. The mole ratio of the lipids present in the lipid mixtures of the compositions of the present invention are:
1. a first lipid mixture having phosphatidylcholines: negatively-charged lipids: sterol compounds in the molar ratio of 1-20:1-10:0.5-5;
2. a second lipid mixture having phosphatidylcholines: negatively-charged lipids: albumin compounds in the molar ratio of 1-20:1-10:0.1-10;
3. a third lipid mixture having phosphatidylcholines: positively-charged lipids: phosphatidylethanolamines in the molar ratio of 1-20:0.5-20:1-10;
4. a fourth lipid mixture having phosphatidylcholines: negatively-charged lipids: phosphatidylethanolamines in the molar ratio of 1-20:1-10:1-10;
5. a fifth lipid mixture having phosphatidylcholines: positively-charged lipids: albumin compounds in the molar ratio of 1-20:0.5-20:0.1-10; or
6. a sixth lipid mixture having phosphatidylcholines: positively-charged lipids: sterol compounds in the molar ratio of 1-20:0.5-20:0.5-5.

Lipid mixtures of the present invention also include:
(i) phosphatidylcholines: negatively-charged lipids: sterol compounds: phosphatidylethanolamines of the molar ratios 1-20:1-10:0.5-5:1-10;
(ii) phosphatidylcholines: negatively-charged lipids: sterol compounds: albumin compounds in the molar ratio of 1-20:1-10:0.5-5:0.1-10;
(iii) phosphatidylcholines: negatively-charged lipids: sterol compounds: phosphatidylethanolamines: albumin compounds in the molar ratio of 1-20:1-10:0.5-5:1-10:0.1-10;
(iv) phosphatidylcholines: negatively-charged lipids: albumin compounds: sterol compounds in the molar ratio of 1-20:1-10:0.1-10:0.5-5;
(v) phosphatidylcholines: positively-charged lipids: phosphatidylethanolamines: sterol compounds in the molar ratio of 1-20:0.5-20:1-10:0.5-5;
(vi) phosphatidylcholines: positively-charged lipids: phosphatidylethanolamines: albumin compounds in the molar ratio of 1-20:0.5-20:1-10:0.1-10;
(vii) phosphatidylcholines: positively-charged lipids: phosphatidylethanolamines: sterol compounds: albumin compounds in the molar ratio of 1-20:0.5-20:1-10:0.5-5:0.1-10; or
(viii) phosphatidylcholines: positively-charged lipids: albumin compounds: sterol compounds in the molar ratio of 1-20:0.5-20:0.1-10:0.5-5.

The bioactive agent: lipid mixture weight ratio can vary from 10 : 1 to 1 : 500. The lipid mixtures when combined with bioactive agents are effective in producing formulations that can be used in inhalation device for administration to the lung. The lipids of a class which is present in the lipid mixture can be a single member of the class or two or more members of the class. Thus, for example the phosphatidylcholines can be a single PC such as DPPC or DPPC with one or more other PC's such as DMPC. The negatively-charged lipids can be PG, PA, PS or PI.

A specific example of lipid mixtures for the composition of the present invention is DPPC : DMPG : cholesterol at a 8.5 : 1.0 : 0.5 mole ratio. Another example can be DPPC : DMPG : albumin at a molar ratio of 8 : 1 : 2.

Another specific lipid mixture is DPPC : DOPE : DMPG : cholesterol at a mole ratio of 7 : 4 : 3 : 0.5.

In general, PE's such as DOPE, DMPE, DPPE, DSPE and MOPE can be employed in the lipid mixtures of the present invention.

For lipid mixtures, particularly for use with biologically active compounds of high molecular weight (e.g., peptides, proteins, DNA, RNA, genes), a glycoprotein such as albumin or transferrin, referred to as an "albumin compound" can be present. The albumin compounds can be present at a mole ratio of 0.1 to 10 with respect to the other lipids. For example, a lipid mixture can be DPPC : DMPG : albumin in a 8 : 1 : 2 mole ratio. The albumin can come from either natural, animal (e.g., human or bovine serum albumin) or synthetic sources. Another example of a lipid mixture of the present invention is DOTAP : DPPC : DOPE present in a mole ratio of 2 : 2 : 1. Other examples of a lipid mixture are DPPC : DOTAP : albumin in a molar ratio of 2:2:0.3; and DPPC : DOTAP : cholesterol in a molar ratio of 8:4:0.5.

The bioactive agents can be a synthetic, semi-synthetic or naturally occurring compounds and include hydrophobic and hydrophilic compounds, compounds of low and high molecular weight (for example from 50 to 100 million Daltons), known drugs, therapeutics, peptides, proteins, DNAs, RNas, genes, and the like.

The present invention includes lipid mixtures in which the molar ratio for any lipid component can be reduced by one-half its value.

A "medical condition" is an illness, infection, physical or mental state, disease, inflammation or other medically-recognized disorder or syndrome. Examples of a medical condition include, but are not limited to, cancer, infection (including but not limited to bacterial, gram negative, mycobacterium, fungal, viral, AIDS, HIV and RSV), need for immune response suppression, prevention of coagulation, thrombosis or adverse platelet aggregation, need for a hormone, diabetes, osteoporosis, hypercalcimia, Paget's Disease, asthma, COPD, cardiovascular disease, hypertension, cardiac arrhythmia, inflammatory disease, pain, nausea, vomiting, smoking addiction, anaphylaxis, cystic fibrosis, Pneumocytyis carnii infection, tuberculosis and emphysema.

Liposomes are completely closed lipid bilayer membranes containing an entrapped aqueous volume. Liposomes may be unilamellar vesicles (possessing a single membrane bilayer) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "heads" orient towards the aqueous phase.

Liposomes can be produced by a variety of methods (for a review, see, e.g., Cullis et al. (1987)). Bangham's procedure (J. Mol. Biol. (1965)) produces ordinary multilamellar vesicles (MLVs). Lenk et al. (U.S. Pat. Nos. 4,522,803, 5,030,453 and 5,169,637), Fountain et al. (U.S. Pat. No. 4,588,578) and Cullis et al. (U.S. Pat. No. 4,975,282) disclose methods for producing multilamellar liposomes having substantially equal interlamellar solute distribution in each of their aqueous compartments. Paphadjopoulos et al., U.S. Pat. No. 4,235,871, discloses preparation of oligolamellar liposomes by reverse phase evaporation.

Unilamellar vesicles can be produced from MLVs by a number of techniques, for example, the extrusion of Cullis et al. (U.S. Pat. No. 5,008,050) and Loughrey et al. (U.S. Pat. No. 5,059,421)). Sonication and homogenization cab be so used to produce smaller unilamellar liposomes from larger liposomes (see, for example, Paphadjopoulos et al. (1968); Deamer and Uster (1983); and Chapman et al. (1968)).

The original liposome preparation of Bangham et al. (J. Mol. Biol., 1965, 13:238-252) involves suspending phospholipids in an organic solvent which is then evaporated to dryness leaving a phospholipid film on the reaction vessel. Next, an appropriate amount of aqueous phase is added, the 60 mixture is allowed to "swell", and the resulting liposomes which consist of multilamellar vesicles (MLVs) are dispersed by mechanical means. This preparation provides the basis for the development of the small sonicated unilamellar vesicles described by Papahadjopoulos et al. (Biochim. Biophys, Acta., 1967, 135:624-638), and large unilamellar vesicles.

Techniques for producing large unilamellar vesicles (LUVs), such as, reverse phase evaporation, infusion procedures, and detergent dilution, can be used to produce liposomes. A review of these and other methods for producing liposomes may be found in the text Liposomes, Marc Ostro, ed., Marcel Dekker, Inc., New York, 1983, Chapter 1, the pertinent portions of which are incorporated herein by reference. See also Szoka, Jr. et al., (1980, Ann. Rev. Biophys. Bioeng., 9:467), the pertinent portions of which are also incorporated herein by reference.

Other techniques that are used to prepare vesicles include those that form reverse-phase evaporation vesicles (REV), Papahadjopoulos et al., U.S. Pat. No. 4,235,871. Another class of liposomes that may be used are those characterized as having substantially equal lamellar solute distribution. This class of liposomes is denominated as stable plurilamellar vesicles (SPLV) as defined in U.S. Pat. No. 4,522,803 to Lenk, et al. and includes monophasic vesicles as described in U.S. Pat. No. 4,588,578 to Fountain, et al. and frozen and thawed multilamellar vesicles (FATMLV) as described above.

A variety of sterols and their water soluble derivatives such as cholesterol hemisuccinate have been used to form liposomes; see specifically Janoff et al., U.S. Pat. No. 4,721,612, issued Jan. 26, 1988, entitled "Steroidal Liposomes." Mayhew et al., PCT Publication No. WO 85/00968, published Mar. 14, 1985, described a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., PCT Publication No. 87/02219, published Apr. 23, 1987, entitled "Alpha Tocopherol-Based Vesicles".

In a liposome-drug delivery system, a bioactive agent such as a drug is entrapped in the liposome and then administered to the patient to be treated. For example, see Rahman et al., U.S. Pat. No. 3,993,754; Sears, U.S. Pat. No. 4,145,410; Paphadjopoulos et al., U.S. Pat. No. 4,235,871; Schneider, U.S. Pat. No. 4,224,179; Lenk et al., U.S. Pat. No. 4,522,803; and Fountain et al., U.S. Pat. No. 4,588,578. Alternatively, if the bioactive agent is lipophilic, it may associate with the lipid bilayer. In the present invention, the term "entrapment" shall be taken to include both the drug in the aqueous volume of the liposome as well as drug associated with the lipid bilayer.

A liposome's "size" is typically referred to in terms of its diameter, and can be measured by a number of techniques well known to ordinarily skilled artisans, such as quasi-electric light scattering. In the present invention the liposomes generally have a diameter of greater than about 1 micron and up to 5 microns, preferably greater than 1 to 3 microns.

Liposomes sizing can be accomplished by a number of methods, such as extrusion, sonication and homogenization techniques which are well known, and readily practiced, by ordinarily skilled artisans. Extrusion involves passing liposomes, under pressure, one or more times through filters having defined pore sizes. The filters are generally made of polycarbonate, but the filters may be made of any durable material which does not interact with the liposomes and which is sufficiently strong to allow extrusion under sufficient pressure. Preferred filters include "straight through" filters because they generally can withstand the higher pressure of the preferred extrusion processes of the present invention. "Tortuous path" filters may also be used. Extrusion can also use asymmetric filters, such as Anotec® filters (see Loughrey et al., U.S. Pat. No. 5,059,421), which involves extruding liposomes through a branched-pore type aluminum oxide porous filter.

Liposomes can also be size reduced by sonication, which employs sonic energy to disrupt or shear liposomes, which will spontaneously reform into smaller liposomes. Sonication is conducted by immersing a glass tube containing the liposome suspension into the sonic epicenter produced in a bath-type sonicator. Alternatively, a probe type sonicator may be used in which the sonic energy is generated by vibration of a titanium probe in direct contact with the liposome suspension. Homogenization and milling apparatii, such as the Gifford Wood homogenizer, Polytron^{™} or Micro fluidizer^{™}, can also be used to break down larger liposomes into smaller liposomes.

The resulting liposomes can be separated into homogeneous populations using methods well known in the art; such as tangential flow filtration (see WO 89/00846). In this procedure, a heterogeneously sized population of liposomes is passed through tangential flow filters, thereby resulting in a liposome population with an upper and/or lower size limit. When two filters of differing sizes, that is, having different pore diameters, are employed, liposomes smaller than the first pore diameter pass through the filter. This filtrate can the be subject to tangential flow filtration through a second filter, having a smaller pore size than the first filter. The retentate of this filter is a liposome population having upper and lower size limits defined by the pore sizes of the first and second filters, respectively.

Mayer et al. found that the problems associated with efficient liposomal entrapment of lipophilic ionizable bioactive agents such as antineoplastic agents, for example, anthracyclines or vinca alkaloids, can be alleviated by employing transmembrane ion gradients (see PCT application 86/01102, published Feb. 27, 1986). Aside from inducing greater uptake, such transmembrane gradients also act to increase drug retention in the liposomes.

Liposomes themselves have been reported to have no significant toxicities in previous human clinical trials where they have been given intravenously. Richardson et al., (1979), Br. J. Cancer 40:35; Ryman et al., (1983) in "Targeting of Drugs" G. Gregoriadis, et al., eds. pp 235-248, Plenum, N.Y.; Gregoriadis G., (1981), Lancet 2:241, and Lopez-Berestein et al., (1985). Liposomes are reported to concentrate predominately in the reticuloendothelial organs lined by sinosoidal capillaries, i.e., liver, spleen, and bone marrow, and phagocytosed by the phagocytic cells present in these organs.

The therapeutic properties of many bioactive agents can be dramatically improved by the administration in a liposomally encapsulated form (See, for example, Shek and Barber (1986)). Toxicity can be reduced, in comparison to the free form of the drug, meaning that a higher dose of the liposomally encapsulated drug can safely be administered (see, for example, Lopez-Berestein, et al. (1985) J. Infect. Dis., 151:704; and Rahman, et al. (1980) Cancer Res., 40:1532). Benefits obtained from liposomal encapsulation likely result from the altered pharmacokinetics and biodistribution of the entrapped drug.

A number of methods are presently available for "charging" liposomes with bioactive agents (see, for example, Rahman et al., U.S. Pat. No. 3,993,754; Sears, U.S. Pat. No. 4,145,410; Papahadjopoulos, et al., U.S. Pat. No. 4,235,871; Lenk et al., U.S. Pat. No. 4,522,803; and Fountain et al., U.S. Pat. No. 4,588,578). Ionizable bioactive agents have been shown to accumulate in liposomes in response to an imposed proton or ionic gradient (see, Bally et al., U.S. Pat. No. 5,077,056; Mayer, et al. (1986); Mayer, et al. (1988); and Bally, et al. (1988)). Liposomal encapsulation could potentially provide numerous beneficial effects for a wide variety of bioactive agents and a high bioactive agent to lipid ratio should prove important in realizing the potential of liposomally encapsulated agents.

For the references disclosed herein, their disclosures are incorporated herein by reference.

A "lipid complex" is an association between a bioactive agent and one or more lipids. The association can be by covalent or ionic bonding or by noncovalent interactions. Examples of such complexes include lipid complexes of amphotericin β and cardiolipin complexed with doxorubicin.

A "lipid clathrate" is a three-dimensional, cage-like structure employing one or more lipids wherein the structure entraps a bioactive agent.

"Proliposomes" are formulations that can become liposomes upon coming in contact with an aqueous liquid. Agitation or other mixing can be necessary.

The inhalation delivery device of the inhalation system can be a nebulizer, a metered dose inhaler (MDI) or a dry powder inhaler (DPI). The device can contain and be used to deliver a single dose of the lipid mixed - bioactive agent compositions or the device can contain and be used to deliver multi-doses of the compositions of the present invention.

A nebulizer type inhalation delivery device can contain the compositions of the present invention as a solution, usually aqueous, or a suspension. In generating the nebulized spray of the compositions for inhalation, the nebulizer type delivery device may be driven ultrasonically, by compressed air, by other gases, electronically or mechanically. The ultrasonic nebulizer device usually works by imposing a rapidly oscillating waveform onto the liquid film of the formulation via an electrochemical vibrating surface. At a given amplitude the waveform becomes unstable, whereby it disintegrates the liquids film, and it produces small droplets of the formulation. The nebulizer device driven by air or other gases operates on the basis that a high pressure gas stream produces a local pressure drop that draws the liquid formulation into the stream of gases via capillary action. This fine liquid stream is then disintegrated by shear forces. The nebulizer may be portable and hand held in design, and may be equipped with a self contained electrical unit. The nebulizer device can consist of a nozzle that has two coincident outlet channels of defined aperture size through which the liquid formulation can be accelerated. This results in impaction of the two streams and atomization of the formulation. The nebulizer may use a mechanical actuator to force the liquid formulation through a multiorifice nozzle of defined aperture size(s) to produce an aerosol of the formulation for inhalation. In the design of single dose nebulizers, blister packs containing single doses of the formulation may be employed.

A metered dose inhalator (MDI) can be employed as the inhalation delivery device of the inhalation system. This device is pressurized (pMDI) and its basic structure consists of a metering valve, an actuator and a container. A propellant is used to discharge the formulation from the device. The composition can consist of particles of a defined size suspended in the pressurized propellant(s) liquid, or the composition can be in a solution or suspension of pressurized liquid propellant(s). The propellants used are primarily atmospheric friendly hydroflourocarbons (HFCs) such as 134a and 227. Traditional chloroflourocarbons like CFC-11, 12 and 114 are used only when essential. The device of the inhalation system may deliver a single dose via, e.g., a blister pack, or it may be multi dose in design. The pressurized metered dose inhalator of the inhalation system can be breath actuated to deliver an accurate dose of the lipid based formulation. To insure accuracy of dosing, the delivery of the formulation may be programmed via a microprocessor to occur at a certain point in the inhalation cycle. The MDI may be portable and hand held.

A dry powder inhalator (DPI) can be used as the inhalation delivery device of the inhalation system. This device's basic design consists of a metering system, a powdered composition and a method to disperse the composition. Forces like rotation and vibration can be used to disperse the composition. The metering and dispersion systems may be mechanically or electrically driven and may be microprocessor programmable. The device may be portable and hand held. The inhalator may be multi or single dose in design and use such options as hard gelatin capsules, and blister packages for accurate unit doses. The composition can be dispersed from the device by passive inhalation; i.e., the patient's own inspiratory effort, or an active dispersion system may be employed. The dry powder of the composition can be sized via processes such as jet milling, spray dying and supercritical fluid manufacture. Acceptable excipients such as the sugars mannitol and maltose may be used in the preparation of the powdered formulations. These are particularly important in the preparation of freeze dried liposomes and lipid complexes. These sugars help in maintaining the liposome's physical characteristics during freeze drying and minimizing their aggregation when they are administered by inhalation. The sugar by its hydroxyl groups may help the vesicles maintain their tertiary hydrated state and help minimize particle aggregation.

These three general types of inhalation delivery devices can also be used to deliver the vaccine compositions of the present invention.

Depending upon the biological activity of the bioactive agents present in the lipid-bioactive agent compositions, the inhalation system of the present invention can be used for the treatment of diseases in a number of therapeutic areas. These therapeutic areas include: infectious disease (anti-bacterial, anti-fungal and anti-viral activity), inflammatory disease (including arthritis, and hypertension), neoplastic disease (cancer), diabetes, osteoporosis, pain management and general cardiovascular disease. As discussed above the inhalation system may also be used in the treatment of lung disease. Lung disease includes: asthma, emphysema, lung cancer, chronic obstructive pulmonary disease (COPD), bronchitis, influenza, pneumonia, tuberculosis, respiratory distress syndrome, cystic fibrosis, sudden infant death syndrome (SDS), respiratory synctial virus (RSV), AIDS related lung diseases (e.g., Pneumocystis carinii pneumonia, Mycobacterium avium complex, fungal infections, etc.), sarcoidosis, sleep apnea, acute respiratory distress syndrome (ARDS), bronchiectasis, bronchiolitis, bronchopulmonary dysplasia, coccidioidomycosis, hantavirus pulmonary syndrome, histoplasmosis, pertussis and pulmonary hypertension.

Some specific examples of bioactive agents that can be present in the compositions of the inhalation system and the uses of the system in the treatment of disease include: sulfonamide, such as sulfonamide, sulfamethoxazole and sulfacetamide; trimethoprim, particularly in combination with sulfamethoxazole; a quinoline such as norfloxacin and ciprofloxacin; a beta- lactam compound including a penicillin such as penicillin G, penicillin V, ampicillin, amoxicillin, and piperacillin, a cephalosporin such as cephalosporin C, cephalothin, cefoxitin and ceftazidime, other beta-lactam antibiotics such as imipenem, and aztreonam; a beta lactamase inhibitor such as clavulanic acid; an aminoglycoside such as gentamycin, amikacin, tobramycin, neomycin, kanamycin and netilmicin; a tetracycine such as chlortetracycline and doxycycline; chloramphenicol; a macrolide such as erythromycin; or miscellaneous antibiotics such as clindamycin, a polymyxin, and bacitracin for anti-bacterial, and in some cases antifungal, infections; a polyene antibiotic such as amphotericin B, nystatin, and hamycin; flucytosine; an imidazole or a triazole such as ketoconazole, miconazole, itraconazole and fluconazole; griseofulvin for anti-fungal diseases such as aspergillosis, candidaisis or histoplasmosis; zidovudine, acyclovir, ganciclovir, vidarabine, idoxuridine, trifluridine, an interferon (e.g, interferon alpha-2a or interferon alpha-2b) and ribavirin for anti-viral disease; aspirin, phenylbutazone, phenacetin, acetaminophen, ibuprofen, indomethacin, sulindac, piroxicam, diclofenac; gold and steroidal anti-inflammatories for inflammatory diseases such as arthritis; an ACE inhibitor such as captopril, enalapril, and lisinopril; the organo nitrates such as amyl nitrite, nitroglycerin and isosorbide dinitrate; the calcium channel blockers such as diltiazem, nifedipine and verapamil; the beta adrenegic antagonists such as propranolol for cardiovascular disease; a diuretic such as a thiazide; e.g., benzothiadiazine or a loop diuretic such as furosemide; a sympatholytic agent such as methyldopa, clonidine, gunabenz, guanaethidine and reserpine; a vasodilator such as hydalazine and minoxidil; a calcium channel blocker such as verapimil; an ACE inhibitor such as captopril for the treatment of hypertension; quinidine, procainamide, lidocaine, encainide, propranolol, esmolol, bretylium, verapimil and diltiazem for the treatment of cardiac arrhythmia; lovostatin, lipitor, clofibrate, cholestryamine, probucol, and nicotinic acid for the treatment of hypolipoproteinemias; an anthracycline such as doxorubicin, daunorubicin and idarubicin; a covalent DNA binding compound, a covalent DNA binding compound and a platinum compound such as cisplatin and carboplatin; a folate antagonist such as methotrexate and trimetrexate; an antimetabolite and a pyrimidine antagonist such as fluorouracil, 5-fluorouracil and fluorodeoxyuridine; an antimetabolite and a purine antagonist such as mercaptopurine, 6-mercaptopurine and thioguanine; an antimetabolite and a sugar modified analog such as cytarabine and fludarabine; an antimetabolite and a ribonucleotide reductase inhibitor such as hydoxyurea; a covalent DNA binding compound and a nitrogen mustard compound such as cyclophosphamide and ifosfamide; a covalent DNA binding compound and an alkane sulfonate such as busulfane; a nitrosourea such as carmustine; a covalent DNA binding compound and a methylating agent such as procarbazine; a covalent DNA binding compound and an aziridine such as mitomycin; a non covalent DNA binding compound; a non covalent DNA binding compound such as mitoxantrone and, bleomycin; an inhibitor of chromatin function and a topoisomerase inhibitor such as etoposide, teniposide, camptothecin and topotecan; an inhibitor of chromatin function and a microtubule inhibitor such as the vinca alkaloids including vincristine, vinblastin, vindisine, and paclitaxel, taxotere or another taxane; a compound affecting endocrine function such as prednisone, prednisolone, tamoxifen, leuprolide, ethinyl estradiol, an antibody such as herceptin; a gene such as the p-53 gene, the p 16 gene, the FHIT gene, and the gene E-cadherin; a cytokine such as the interleukins, particularly, IL-1, IL-2, IL-4, IL-6, IL-8 and IL-12, the tumor necrosis factors such as tumor necrosis factor- alpha and tumor necrosis factor-beta, the colony stimulating factors such as granulocyte colony stimulating factor (G-CSF), macrophage colony stimulating factor (M-CSF) and, granulocyte macrophage colony stimulating factor (GM-CSF) an interferon such as interferon-alpha, interferon -beta 1, interferon-beta 2, and interferon-gamma; all-trans retinoic acid or another retinoid for the treatment of cancer; an immunosupressive agent such as: cyclosporine, an immune globulin, and sulfasazine, methoxsalen and thalidoimide; insulin and glucogon for diabetes; calcitonin and sodium alendronate for treatment of osteoporosis, hypercalcemia and Paget's Disease; morphine and related opioids; meperidine or a congener; methadone or a congener; an opioid antagonist such as nalorphine; a centrally active antitussive agent such as dexthromethrophan; tetrahydrocannabinol or marinol, lidocaine and bupivicaine for pain management; chloropromazine, prochlorperazine; a cannabinoid such as tetrahydrocannabinol, a butyrophenone such as droperidol; a benzamide such as metoclopramide for the treatment of nausea and vomiting; heparin, coumarin, streptokinase, tissue plasminogen activator factor(t-PA) as anticoagulant, antithrombolytic or antiplatelet drugs; heparin, sulfasalazine, nicotine and adrenocortical steroids and tumor necrosis factor- alpha for the treatment of inflammatory bowel disease; nicotine for the treatment of smoking addiction; growth hormone, luetinizing hormone, corticotropin, and somatotropin for hormonal therapy; and adrenaline for general anaphylaxis.

In regard to lung disease, bioactive agents that can be present in the compositions of the inhalation system and the uses of the system in the treatment of disease include: a methylxanthine such as theophylline; cromolyn; a beta- adrenginic agonist such as albuterol and tetrabutaline; a anticholinergic alkaloid such as atropine and ipatropium bromide; adrenocortical steroids such as predisone, beclomethasone and dexamethasone for asthma or inflammatory disease; the anti-bacterial and antifungal agents listed above for anti- bacterial and anti-fungal infections in patients with lung disease (these are the specific diseases listed above in what lung disease includes), in particular this includes the use of aminoglycosides (e.g., amikacin, tobramycin and gentamycin), polymyxins (e.g., polymyxin E, colistin), carboxycillin (ticarcillin) and monobactams for the treatment of gram- negative anti-bacterial infections, for example, in cystic fibrosis patients, for the treatment of gram negative infections of patients with tuberculosis, for the treatment of gram negative infections in patients with chronic bronchitis and bronchiectasis, and for the treatment of gram negative infections in generally immuno-compromised patients; the use of pentamidine for the treatment of patients (e.g., HIV/AIDS patients) with Pneumocytis carinii infections; the use of a polyene antibiotic such as amphotericin B, nyststin, and hamycin; flucytosine; an imidazole or a triazole such as ketoconazole, miconazole, itraconazole and fluconazole; griseofulvin for the treatment of such fungal infections as aspergillosis, candidiasis and histoplasmosis, particularly those originating or diseminating to the lungs; the use of the corticosteroids and other steroids as listed above, as well as nonsteroidal anti-inflammatory drugs for the treatment of anti-inflammatory conditions in patients with lung disease (these are the specific diseases listed above in what lung disease includes); DNase, amiloride, CFTRcDNA in the treatment of cystic fibrosis; alpha-1-antitrypsin and alpha-1-antitrypsin cDNA for the treatment of emphysema; an aminoglycoside such as amikacin, tobramycin or gentamycin, isoniazid, ethambutol, rifampin and its analogs for the treatment of tuberculosis or mycobacterium infections; ribavirin for the treatment of respiratory synctial virus; the use of the anticancer agents listed above for lung cancer in particular cisplatin, carboplatin, and taxanes such as paclitaxel, and the taxanes, camptothecin, topotecin, and other camptothecins, herceptin, the p-53 gene and IL-2.

The pharmaceutical formulation of the inhalation system may contain more than one pharmaceutical (e.g., two drugs for a synergistic effect).

In addition to the above discussed lipids and albumin and drug(s), the composition of the pharmaceutical formulation of the inhalation system may contain excipients(including solvents, salts and buffers), preservatives and surfactants that are acceptable for administration by inhalation to humans or animals.

The particle size of the pharmaceutical formulations developed for use in the inhalation system can vary and be between 0.5 and 10 microns, with a range of 1 to 5 microns being best suited for inhalation. The particle size of the formulations can be established either prior to their filling into the inhalation delivery device of the inhalation system; or depending upon its design, the inhalation delivery device may size the particles. The pharmaceutical formulation of the inhalation system may be present as a powder, a liquid, or as a suspension.

The inhalation system can also be used for the administration of vaccines. In this case, the vaccine's composition would consist of the lipids discussed above in the presented mole composition ratios and a biologically active antigen(s). The lipid: antigen mole ratio could be from 1,000,000 : 1 to 1 : 100. The inhalation system would consist of the lipid based vaccine formulation and the inhalation delivery device. The vaccine inhalation systems may be used in the preventive (i.e., prophylactic) and actual treatment of the diseases described above, particularly lung diseases like, influenza, pneumonia, RSV, tuberculosis, etc. The particle size, and how it is produced is as discussed above, and the vaccine formulation may contain excipients(including solvents, salts and buffers), preservatives and surfactants that are acceptable for administration by inhalation to humans or animals. The vaccine formulation could contain more than one antigen.

The term "treatment" or "treating" means administering a composition to an animal such as a mammal or human for preventing, ameliorating, treating or improving a medical condition.

In general, the doses of a bioactive agent will be chosen by a physician based on the age, physical condition, weight and other factors known in the medical arts. Generally for bioactive agents, the dosages will be within the same employing the present invention as for the free drug.

### REFERENCES FOR INHALATION DEVICE

1. Hickey A.J. and Dunbar C.A., " A New Millennium for Inhaler Technology", Pharmaceutical Technology, pgs. 119-125, June (1997).
2. P. Lloyd et. al., " A New Unit Dose, Breath- Actuated Aerosol Drug Delivery System," in Respiratory Drug Delivery V, P.R. Byron, R.N. Dalby and S.J. Farr, Eds., Interpharm. Press, Buffalo Grove, pgs. 364-366 (1996).
3. J.H. Bell, P.S. Hartley and J.S.G. Cox, " Dry Powder Aerosols I: A New Inhalation Device," J. Pharm. Sci., 60 (10), pgs. 1539-1564 (1971).
4. J.W. Tom and P.G. Debendetti, " Particle Formation with Supercritical Liquids- A Review," J. Aerosol Sci., 22, pgs. 555-584 (1991).
5. M.P. Billings, R.N. Boyes, L.M. Clisby, A.E. Harper, P. Braithwaite and S. Williams, " Design, Development and Performance of a Novel Multidose Dry Powder Inhaler," Pharmaceutical Technology, pgs. 70-82, October (1999).
6. Ogden J., Rogerson C. and Smith I., "Issues in Pulmonary Delivery", Scripps Magazine, pgs. 56-60, June (1997).

### OTHER REFERENCES

1. Possmayer F.I., Metcalfe I.L. and Ehorning G., The Pulmonary Surfactant-Contol of Fluidity at the Air-Liquid Membrane, In:: Membrane Fluidity, Kates M. and Katis A. (eds.), pgs. 57-67, Clifton NJ, Humana Press (1980).
2. Ligand Targeting of Liposomes, Leserman L. and Machy P., In:: Liposomes from Biophysics to Therapeutics, pgs 157-194, Ostro M. (ed.), New York, NY, Marcel Dekker (1987).
3. Gonzalez-Rothi R.J., Casace J., Straub L. and Schreier H., Liposomes and Pulmonary Alveolar Macrophages: Functional and Morphologic Interactions, Exp. Lung Res., 17, pgs. 687-705 (1991).
4. Geiger K., Gallagher M.L. and Hedley -White J., Cellular Distribution and Clearance of Aerosolized Dipalmitoyl Choline, J. Appl. Physiol., 39, pgs. 759-766 (1975).
5. Bates S. R., Ibach P.B. and Fisher A. B., Phospholipids Co-Isolated with Rat Protein C Account for the Apparent Protein Enhanced Uptake of Liposomes into Lung Granular Pneumocytes, Exp. Lung Res., 15, pgs. 695-708 (1989).
6. A.G. Goodman, and L. Goodman " The Pharmacological Basis of Therapeutics, Eighth Edition" A.G. Goodman, T.W. Rall, A.S. Nies and P. Taylor, Editors, Permagon Press, New York, NY (1990).

## Claims

1. A system for administering a bioactive compound by inhalation comprising:
(a) a composition comprising the biologically-active compound and a lipid mixture; and
(b) an inhalation delivery device;
wherein the lipid mixture comprises:
(1) a first mixture comprising phosphatidylcholine, negatively-charged lipid, and sterol in the molar ratio of 1-20:1-10:0.5-5;
(2) a second mixture comprising phosphatidylcholine, negatively-charged lipid and albumin in the molar ratio of 1-20:1-10:0.1-10;
(3) a third mixture comprising phosphatidylcholine, positively-charged lipid and phosphatidylethanolamine in the molar ratio of 1-20:0.5-20:1-10;
(4) a fourth lipid mixture having phosphatidylcholines: negatively-charged lipids: phosphatidylethanolamines in the molar ratio of 1-20:1-10:1-10;
(5) a fifth lipid mixture having phosphatidylcholines: positively-charged lipids: albumin compounds in the molar ratio of 1-20:0.5-20:0.1-10; or
(6) a sixth lipid mixture having phosphatidylcholines: positively-charged lipids: sterol compounds in the molar ratio of 1-20:0.5-20:0.5-5.
wherein the bioactive agent lipid mixture weight ratio is from 10:1 to 1:500.

2. The system of Claim 1 wherein the lipid mixture comprises:
(i) phosphatidylcholines: negatively-charged lipids: sterol compounds: phosphatidylethanolamines of the molar ratios 1-20:1-10:0.5-5:1-10;
(ii) phosphatidylcholines: negatively-charged lipids: sterol compounds: albumin compounds in the molar ratio of 1-20:1-10:0.5-5:0.1-10;
(iii) phosphatidylcholines: negatively-charged lipids: sterol compounds: phosphatidylethanolamines: albumin compounds in the molar ratio of 1-20:1-10:0.5-5:1-10:0.1-10;
(iv) phosphatidylcholines: negatively-charged lipids: albumin compounds: sterol compounds in the molar ratio of 1-20:1-10:0.1-10:0.5-5;
(v) phosphatidylcholines: positively-charged lipids: phosphatidylethanolamines: sterol compounds in the molar ratio of 1-20:0.5-20:1-10:0.5-5;
(vi) phosphatidylcholines: positively-charged lipids: phosphatidylethanolamines: albumin compounds in the molar ratio of 1-20:0.5-20:1-10:0.1-10;
(vii) phosphatidylcholines: positively-charged lipids: phosphatidylethanolamines: sterol compounds: albumin compounds in the molar ratio of 1-20:0.5-20:1-10:0.5-5:0.1-10; or
(viii) phosphatidylcholines: positively-charged lipids: albumin compounds: sterol compounds in the molar ratio of 1-20:0.5-20:0.1-10:0.5-5.

3. The system of Claim 1 wherein the negatively-charged lipid is at least one of phosphatidylglycerol, phosphatidic acid or phosphatidylinisotol.

4. The system of Claim 1 wherein the first mixture additionally comprises phosphatidylethanolamine so that the molar ratio of phosphatidylcholine: negatively-charged lipid: sterol: phosphatidylethanolamine is 1-20:1-3:0.5-5:1-10.

5. The system of Claim 3 wherein the first mixture further comprises albumin so that the molar ratio of phosphatidylcholine: negatively-charged lipid: sterol:
phosphatidylethanolamine: albumin is 1-20:1-5:0.5-5:1-10:0.1-10.

6. The system of Claim 1 wherein the first mixture additionally comprises albumin so that the molar ratio of phosphatidylcholine: negatively-charged lipid: sterol: albumin is 1-20:1-5:0.5-5:0.1-10.

7. The system of Claim 1 wherein for the first mixture the molar ratio is 5-10:1-5:0.5-5.

8. The system of Claim 7 wherein the negatively-charged lipid is at least one of phosphatidylglycerol, phosphatidic acid or phosphatidylinisotol.

9. The system of Claim 8 wherein the first mixture comprises DPPC: DPMG: cholesterol: DOPE in the molar ratio of 7:3:0.5:4.

10. The system of Claim 1 wherein the second mixture comprises DPPC: DPPG: albumin in a molar ratio of 8:1:2.

11. The system of Claim 1 wherein the third mixture comprises DPPC: DOTAP: DOPE in the molar ratio of 2:2:1.

12. The system of Claim 1 wherein the second mixture additionally comprises phosphatidylethanolamine so that the molar ratio of phsophatidylcholine: negatively-charged lipid: albumin: phosphatidlethanolamine is 1-20:1-10:0.1-10:1-10.

13. The system of Claim 1 wherein the second mixture further contains sterol so that the molar ratio of phosphatidylcholine: negatively-charged lipid: albumin: phosphatidylethanolamine: sterol is 1-20:1-10:0.1-10:1-10:0.5-5.

14. The system of Claim 1 wherein the second mixture additionally comprises sterol so that the molar ratio of phosphatidylcholine: negatively-charged lipid: albumin: sterol is 1-20:1-10:0.1-10:0.5-5.

15. The system of Claim 1 wherein the third mixture additionally comprises albumin so that the ratio of phosphatidylcholine: positively-charged lipid: phosphatidylethanolamine: albumin is 1-20:0.5-20:1-10:0.1-10.

16. The system of Claim 14 wherein the third mixture additionally comprises sterol so that the ratio of phosphatidylcholine: positively-charged lipid: phosphatidylethanolamine: albumin: sterol is 1-20:0.5-20:1-10:0.1-10:0.5-5.

17. The system of Claim 1 wherein the third mixture additionally comprises sterol so that the ratio of phsophatidylcholine: positively-charged lipid: phosphatidylethanolamine: sterol is 1-20:0.5-20:1-10:0.5-5.

18. The system of Claim 1 wherein the lipid mixture comprises DPPC : DMPG : cholesterol in a 8.5:1.0:0.5 molar ratio.

19. The system of Claim 1 wherein the lipid mixture comprises DPPC : DOPE: DMPG: cholesterol in a 7:4:3:0.5 molar ratio.

20. The system of Claim 1 wherein the lipid mixture comprises DMPC : DMPG : albumin n a 8:1:2 molar ratio.

21. The system of Claim 20 wherein the bioactive agent is a peptide, protein, DNA, RNA or a gene.

22. The system of Claim 1 wherein the lipid mixture comprises DOTAP : DPPC : DOPE in a 2:2:1 molar ratio.

23. Use of the system of any one of claims 1 to 22 for use in the preparation of a medicament for treating a medical condition.

24. Use of claim 23 wherein the medical condition is cancer.

25. Use of claim 24 wherein the bioactive agent is an anthracycline, a platinum compound, a folate antagonist, a pyrimidine antagonist, a purine antagonist, a sugar modified analog, a ribonucleotide reductase inhibitor, a nitrogen mustard compound, an alkane sulfonate, a nitrosourea, a methylating agent, an aziridine, mitoxantrone, a compound affecting endocrine function, a topoisomerase inhibitor, an antibody, a gene, bleomycin, a microtubule inhibitor, a cytokine or a differentiating agent.

26. Use of claim 25 wherein the platinum compound is carboplatin or cisplatin.

27. Use of claim 25 wherein the topoisomerase inhibitor is a camptothecin.

28. Use of claim 25 wherein the differentiating agent is a retinoid.

29. Use of claim 25 wherein the gene is the p-53 gene, the p-16 gene, the FHIT gene, and the gene E-cadherin.

30. Use of claim 29 wherein the gene is the p-53 gene.

31. Use of claim 23 wherein the medical condition is an infection.

32. Use of claim 31 wherein the bioactive agent is an anti-infective.

33. Use of claim 32 wherein the anti-infective is a sulfonamide, trimethoprim, a quinoline, a beta- lactam, a beta-lactamase inhibitor, an aminoglycoside, a tetracycline, chloramphenicol, a macrolide, clindamycin, a polymyxin or bacitracin.

34. Use of claim 33 wherein the aminoglycoside is amikacin or tobramycin.

35. Use of claim 33 wherein the medical condition is a gram negative infection, or a mycobacterium infection.

36. Use of claim 35 wherein the aminoglycoside is amikacin or tobramycin.

37. Use of claim 23 wherein the medical condition is a fungal infection.

38. Use of claim 37 wherein the bioactive agent is a polyene antibiotic, flucytosine, an imidazole, a triazole or griseofulvin.

39. Use of claim 38 wherein the polyene antibiotic is amphotericin B, hamycin or nystatin.

40. Use of claim 38 wherein the imidazole or triazole is ketoconazole or fluconazole.

41. Use of claim 37 wherein the fungal infection is aspergillosis, candidiasis or histoplasmosis.

42. Use of claim 23 wherein the medical condition is a viral infection.

43. Use of claim 42 wherein the bioactive agent is antiviral compound.

44. Use of claim 42 or 43 wherein the viral infection is AIDS or HIV.

45. Use of claim 42 or 43 wherein the viral infection is respiratory synctial virus (RSV).

46. Use of any one of claims 42 to 45 wherein the bioactive agent is zidovudine, acyclovir, ganciclovir, vidarabine, idoxuridine, trifluridine, an interferon or ribavirin.

47. Use of claim 23 wherein the bioactive agent is an immunosupressive agent.

48. Use of claim 47 wherein the immunosupressive agent is cyclosporine, an immune globulin, sulfasazine, methoxsalen or thalidomine.

49. Use of claim 23 wherein the bioactive agent is an anticoagulant, antithrombolytic or antiplatelet drug.

50. Use of claim 49 wherein the bioactive agent is heparin, coumarin, streptokinase, urikinase, tissue plasminogen activator factor(t-PA), aspirin, dipyrimadole or ticlopidine.

51. Use of claim 23 wherein the bioactive compound is a hormone.

52. Use of claim 51 wherein the hormone is growth hormone, luetinizing hormone, corticotropin or somatotropin.

53. Use of claim 23 wherein the medical condition is diabetes.

54. Use of claim 53 wherein the bioactive agent is insulin or glucogon.

55. Use of claim 23 wherein the medical condition is osteoprosis, hypercalcemia or Pagent's Disease.

56. Use of claim 55 wherein the bioactive agent is calcitonin or sodium alendronate.

57. Use of claim 23 wherein the medical condition is asthma.

58. Use of claim 57 wherein the bioactive agent is methylxanthine, cromolyn, a beta- adrenginic agonist, an anticholinergic alkaloid or an adrenocortical steroid.

59. Use of claim 23 wherein the medical condition is chronic obstructive pulmonary disease.

60. Use of claim 59 wherein the bioactive agent is a methylxanthine, cromolyn; a beta- adrenginic agonist, an anticholinergic alkaloid or an adrenocortical steroid.

61. Use of claim 23 wherein the medical condition is cardiovascular disease.

62. Use of claim 61 wherein the bioactive agent is an ACE inhibitor, an organonitrate, a calcium channel blocker or a beta adrenegic antagonist.

63. Use of claim 23 wherein the medical condition is hypertension.

64. Use of claim 63 wherein the bioactive agent is a diuretic, a sympatholytic agent, a vasodilator, a calcium channel blocker or an ACE inhibitor.

65. Use of claim 23 wherein the medical condition is cardiac arrhythmia.

66. Use of claim 65 wherein the bioactive agent is quinidine, procainamide, lidocaine, encainide, propranolol, esmolol, bretylium, verapimil or diltiazem.

67. Use of claim 23 wherein the medical condition is inflammatory disease.

68. Use of claim 67 wherein the bioactive agent is a methylxanthine, cromolyn, a beta-adrenginic agonist, an anticholinergic alkaloid or an adreno corticosteroid.

69. Use of claim 23 wherein the medical condition is pain.

70. Use of claim 69 wherein the bioactive agent is an opioid, meperidine or a congener, methadone or a congener, an opioid antagonist, a centrally active antitussive agent or a cannabinoid.

71. Use of claim 70 wherein the bioavtive agent is tetrahydrocannabinol.

72. Use of claim 23 wherein the medical condition is nausea or vomiting.

73. Use of claim 72 wherein the bioactive agent is chloropromazine, prochlorperazine, a cannabinoid, a butyrophenone or a benzamide.

74. Use of claim 23 wherein the medical condition is smoking addiction.

75. Use of claim 74 wherein the bioactive agent is nicotine.

76. Use of claim 23 wherein the medical condition is anaphylaxis.

77. Use of claim 76 wherein the bioactive agent is adrenaline.

78. Use of claim 23 wherein the medical condition is cystic fibrosis.

79. Use of claim 78 wherein the bioactive agent is Dnase, amiloride or CFTRcDNA.

80. Use of claim 23 wherein the medical condition is Pneumocytis carinii infection.

81. Use of claim 80 wherein the bioactive agent is pentamidine.

82. Use of claim 23 wherein the medical condition is emphysema.

83. Use of claim 82 wherein the bioactive agent is alpha-1-antitrypsin or alpha-1-antitrypsin cDNA.

84. Use of claim 23 wherein the medical condition is a mycobacterium infection.

85. Use of claim 84 wherein the mycobacterium infection is tuberculosis.

86. Use of claim 85 wherein the bioactive agent is isoniazid, ethambutol, rifampin and its analogs or an aminoglycoside.

87. Use of claim 23 wherein the bioactive agent is a vaccine.
